# EUROPEAN PATENT APPLICATION

(11) **EP 3 556 838 A1**
(43) Date of publication of application: **23.10.2019**
(21) Application number: 17881466.1
(22) Date of filing: 10.10.2017
(51) Int. Cl.: C12M 1/00, C12N 5/00

(54) **CELL STRUCTURE PRODUCTION DEVICE AND CELL TRAY**

(30) Priority: 13.12.2016 JP 2016241454
(71) Applicant: Cyfuse Biomedical K. K., Bunkyo-ku, Tokyo 113-0033 (JP)
(72) Inventor: KISHII Yasuto, Tokyo 113-0033 (JP); TOKUNAGA Norihiko, Tokyo 113-0033 (JP)
(74) Representative: Mewburn Ellis LLP
(86) International application number: PCT/JP2017/036668
(87) International publication number: WO 2018/110053

(57) **Abstract**

The cell structure producing apparatus capable of properly piercing a cell aggregate and a cell tray are demanded. The object of the present invention is achieved by a cell structure producing apparatus that includes a movable needle-shaped body and sticks and penetrates a cell aggregate held on a cell tray, wherein the cell tray includes a frame having an opening, and a porous member that is supported at the opening by the frame and on which the cell aggregate is placeable, and a tip of the needle-shaped body is capable of penetrating through the porous member when the needle-shaped body sticks and penetrates the cell aggregate.

## Description

### Technical Field

The present invention relates to a cell structure producing apparatus used for manufacturing a three-dimensional structure of cells and a cell tray.

### Background Art

Conventionally, a technique is known of forming a three-dimensional structure by three-dimensionally stacking a plurality of cell aggregates so as to adjoin each other using a property of the cell aggregates that adjoin and contact each other fusing together. With this technique, the cell aggregates arranged on a culture plate are picked up, and the plurality of cell aggregates held by suction or the like are stuck and penetrated by a plurality of needle-shaped bodies, which are immovably secured to a support so as to vertically extend from the support, to make a set of the cell aggregates skewered into a needle-shaped body. The set of the cell aggregates are brought close contact the next set of the cell aggregates with each other, and after the cell aggregates fuse together, the cell aggregates are drawn from the needle-shaped bodies to obtain a three-dimensional structure of cells. In this respect, PTL 1 discloses a technique of vertically moving a thin needle-shaped body down to cell aggregates immovably arranged on a culture plate to pierce the cell aggregates. PTL 1 discloses a technique in which a cell aggregate is individually placed in a recess provided in a surface of the culture plate to prevent the position of a cell aggregate before sticking from changing, and the needle-shaped body is moved down to the cell aggregate from directly above the recess to pierce the cell aggregate.

### Citation List

### Patent Literature

PTL 1: International Publication No. WO 2016/047737

### Summary of Invention

### Technical Problem

However, PTL 1 is based on the fact that one cell aggregate is housed in one recess, and is not intended to hold a plurality of cell aggregates. In PTL 1, depending on types of the cell aggregate, pressure of a tip of the needle-shaped body may deform, rotate, and/or move the cell aggregate in the recess. If the cell aggregate is deformed, rotated, and/or moved, the needle-shaped body sometimes does not pierce the cell aggregate at a proper position. If the cell aggregate that is not stuck at the proper position is cultured, a three-dimensional structure constituted by a plurality of cell aggregates is sometimes unlikely to have a desired three-dimensional shape. Further, tissue obtained by culturing such a three-dimensional structure is sometimes unlikely to have a proper shape. Also, PTL 1 adopts a technique in which, in order to correctly specify a center of the cell aggregate, a laser beam is applied to the cell aggregate, the laser beam reflected by a cell tray is received, and a position of the cell aggregate is determined according to intensity of the laser beam. However, determination of the position according to the reflected laser beam is low in accuracy to specify the center of the cell aggregate to be stuck by the needle-shaped body.

The present invention is achieved in view of these problems, and has an object to provide a cell structure producing apparatus capable of properly piercing a cell aggregate, and a cell tray used in the cell structure producing apparatus .

### Solution to Problem

The object is achieved by a cell structure producing apparatus for sticking and penetrating a cell aggregate held on a cell tray, comprising a movable needle-shaped body, wherein the cell tray includes a frame having an opening, and a porous member supported in the opening by the frame, the cell aggregate being placeable on the porous member, wherein a tip of the needle-shaped body is capable of penetrating through the porous member when the needle-shaped body sticks and penetrates the cell aggregate.

The object is also achieved by a cell tray for holding a cell aggregate in a cell structure producing apparatus including a movable needle-shaped body, for sticking and penetrating the cell aggregate by the movable needle-shaped body, the cell tray including a frame having an opening, and a porous member supported in the opening by the frame, the cell aggregate being placeable on the porous member, wherein a tip of the needle-shaped body is capable of penetrating through the porous member when the needle-shaped body sticks and penetrates the cell aggregate.

### Advantageous Effects of Invention

With the cell tray of the present invention, the cell structure producing apparatus can easily properly pierce the cell aggregate.

### Brief Description of Drawings

Fig. 1 is a schematic perspective sectional view of a cell tray and a cell tray rack.
Fig. 2 is a schematic block diagram of a cell structure producing apparatus.
Fig. 3 shows a step of piercing cell aggregates.
Fig. 4 shows the step of piercing cell aggregates.
Fig. 5 shows the step of piercing cell aggregates.

### Description of Embodiments

A cell tray is placed in a cell structure producing apparatus to hold cell aggregates. The cell structure producing apparatus includes a piercing portion having a needle-shaped body mounted to extend long and thin. The piercing portion can move the needle-shaped body toward the cell aggregates in an extending direction of the needle-shaped body. The piercing portion uses the needle-shaped body to pierce the cell aggregates held on the cell tray to obtain a plurality of cell aggregates skewered by one needle-shaped body. The cell tray includes a porous member and a frame, and the porous member is supported at an opening provided in the frame. The cell structure producing apparatus includes an imaging unit for specifying a position of a cell aggregate to be stuck, and a control unit that controls the piercing portion according to the specified position. The imaging unit includes a light emitting unit that emits light from one side of the porous member in the cell tray toward the placed cell tray, and a light receiving unit that receives the light emitted from the light emitting unit on the other side of the porous member in the cell tray. The control unit controls a position of the needle-shaped body of the piercing portion based on information from the imaging unit. The light emitting unit and the light receiving unit may be provided on either side of the cell tray. The light emitting unit may be provided on a lower side of the cell tray and the light receiving unit may be provided on an upper side of the cell tray, or vice versa. The light receiving unit may include an imaging device such as a camera to take an image, or may include a light receiving sensor such as an optical sensor. The cell tray includes the porous member and the frame.

The porous member may be made of various materials that the needle-shaped body can easily penetrate. The needle-shaped body is a long and thin member to pierce a fine cell aggregate, and is thus easily elastically deformed, plastically deformed, or broken. Thus, if the porous member is hard, the needle-shaped body is easily deformed when piercing the cell aggregate held on the cell tray. If the needle-shaped body is deformed, the needle-shaped body cannot be accurately directed to the position of the cell aggregate to be stuck. Thus, the porous member has a structure that the needle-shaped body can easily penetrate. The porous member has a plurality of holes or gaps, and at least parts of the holes or gaps are coupled in the porous member in any form. The porous member may be made of various materials such as a foamed material, a non-woven fabric, a woven fabric, a knitted fabric, or fiber of any combinations thereof. The porous member is typically in the form of a sheet, but may be in the form that can be supported at the opening. The opening is typically a through hole, and the porous member is mounted to close the through hole. The porous member such as the foamed material or fiber has the plurality of holes or gaps coupled to each other, and thus the needle-shaped body can easily pass through the coupled holes or gaps and penetrate the porous member. The material itself for the porous member may have high light transmittance, or the porous member having many holes or gaps coupled has high light transmittance through the holes. Thus, the porous member can transmit light emitted from one side of the porous member, and receive the transmitted light on the other side of the porous member. This embodiment will be described below.

### Embodiment

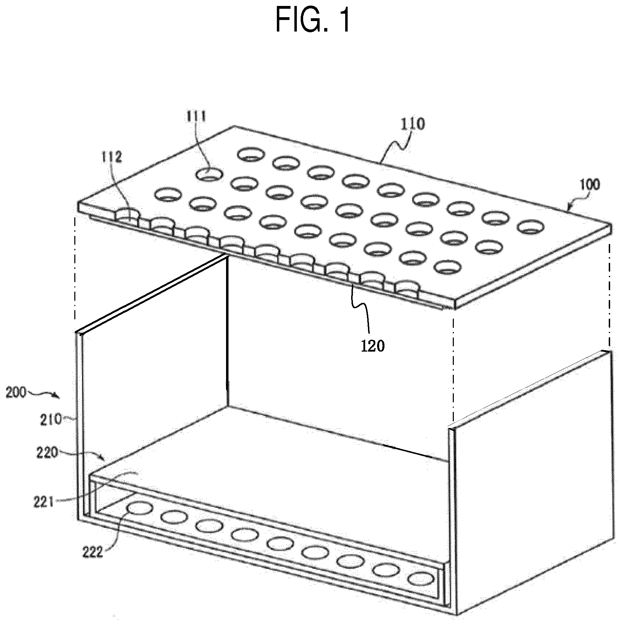

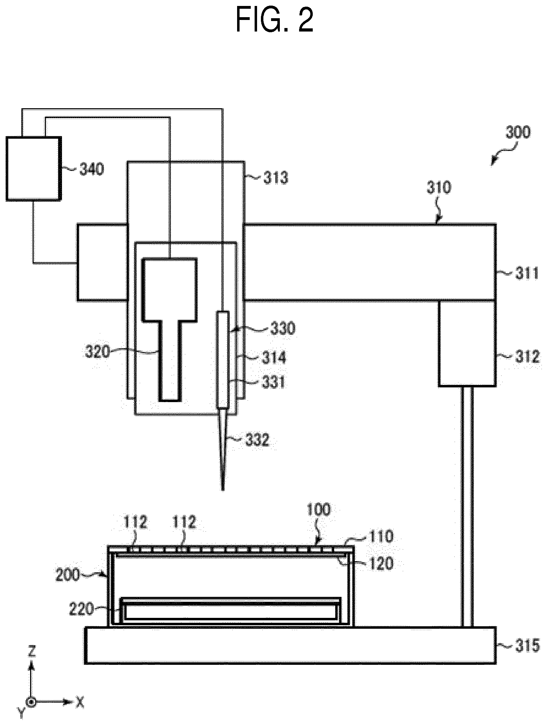

First, with reference to Figs. 1 and 2, a cell tray 100 and a cell structure producing apparatus 300 using the cell tray 100 will be described as an embodiment of the present invention. Fig. 1 shows the cell tray 100 placed on a cell tray rack 200 of the cell structure producing apparatus 300. Fig. 2 shows the cell structure producing apparatus 300 in which the cell tray 100 is placed on the cell tray rack 200.

The cell tray 100 includes a frame 110 and a porous member. In this embodiment, a non-woven fabric sheet 120 is chosen as the porous member by way of example.

The frame 110 is a plate-like member having a plurality of openings 111 extending through the frame 110 in a thickness direction of the frame 110. The frame 110 is formed of, for example, resin such as ABS or metal such as aluminum or stainless, for example, by cutting or die punching. The frame 110 may have any size, and has, for example, a width of 141 millimeters, a depth of 101 millimeters, and a thickness of 2 millimeters. The number and size of the openings 111 may be freely chosen when seen in the thickness direction of the frame 110. The opening 111 is a through hole provided in the thickness direction of the frame 110. For example, 96 cylindrical openings 111 may be provided in an 8-by-12 matrix, and each may have any diameter. For example, the diameter of the hole may be a few millimeters to about 8 millimeters, and a center-to-center distance between adjacent openings 111 is set according to the diameter. The center-to-center distance may be, for example, 9 millimeters for the openings 111 in the 8-by-12 matrix with a diameter of 6 millimeters.

The non-woven fabric sheet 120 is a typical material for the porous member, but various materials may be used as described above. Fiber members such as a non-woven fabric or a woven fabric may be widely used, not limited to the non-woven fabric. Any fibrous materials for the fiber member may be chosen, and the fibrous material may have any diameter. For example, the non-woven fabric sheet 120 is made of a non-woven fabric formed by stacking one or more fibrous materials and then twining fibers or bonding fibers with an adhesive without weaving to form a sheet. The non-woven fabric is made of a mesh-like and/or porous material that a needle-shaped member described later can easily penetrate without being damaged and that can hold a buffer solution or a culture solution and can counteract a force applied by the needle-shaped member described later. The non-woven fabric sheet 120 has, for example, a width of 123 millimeters, a depth of 89 millimeters, and a thickness of 0.2 millimeters. The non-woven fabric sheet 120 is, for example, white, and becomes transparent or nearly transparent and has light transmittance when water is absorbed in it. The buffer solution contains phosphate buffer saline or the like, and the culture solution contains physiologically active substance. The non-woven fabric sheet 120 is attached to one side of the frame 110 and supported at the opening 111. The non-woven fabric sheet 120 may be supported at the opening 111 in any position, and may be supported at an end of the opening 111 or in the opening 111. For example, the non-woven fabric sheet 120 may be attached to one side of the frame 110 near the opening 111 so as to close the opening 111 as a through hole and supported. The non-woven fabric sheet 120 may be supported on a wall surface in the opening 111 to seal the through hole. From the through hole and a surface, to which the non-woven fabric sheet 120 is not attached, of the frame 110 that seals the through hole, a closed-end cylindrical recess 112 with its bottom surface being the non-woven fabric sheet 120 is formed. When a cell aggregate is placed on the non-woven fabric sheet 120, a tip of a needle 332 described later presses the cell aggregate from a side opposite to the non-woven fabric sheet 120, and then the cell aggregate is about to be deformed to be crushed, rotated, and/or moved. In this respect, the non-woven fabric sheet 120 slightly bends and is deformable so as to support the cell aggregate against the pressure of the tip and wrap around the cell aggregate. The non-woven fabric sheet 120 is mainly formed by stacking or twining independent fiber thereon without weaving, and thus generally has lower fiber density than a fabric woven from a plurality of fiber bundles. Thus, effective action of unsolid regions, that is, unblocked regions due to porosity of the non-woven fabric sheet 120 allows the needle 332 to enter the non-woven fabric sheet 120 without any stress, that is, without any reaction force from the non-woven fabric sheet 120. The needle-shaped body is a long and thin member to pierce a fine cell aggregate, and is thus easily elastically deformed, plastically deformed, or broken by a slight load. Thus, if the non-woven fabric sheet 120 is hard, the needle-shaped body is easily deformed when piercing the cell aggregate held on the cell tray 100. If the needle-shaped body is deformed, the needle-shaped body cannot be accurately directed to a position of the cell aggregate to be stuck. Thus, the porous member has a structure that the needle-shaped body can easily penetrate.

The cell tray rack 200 is a seat including a box 210 and a light emitting unit 220 as a camera obscura and on which the cell tray 100 is placed. The box 210 is a rectangular parallelepiped box that does not have a top surface. An inside of the box 210 is surface-treated to be, for example, painted matte black. The box 210 is formed of, for example, resin such as ABS or metal such as aluminum or stainless. The light emitting unit 220 is a rectangular parallelepiped box smaller than the inside of the box 210, and mainly includes a white light transmitting plate 221 provided on a top surface, and a plurality of LEDs 222 stored in the light emitting unit 220 and emits white light. The light emitted from the LEDs 222 are properly diffused by the light transmitting plate 221, and emitted from the top surface of the light emitting unit 220 as even white light. The light emitting unit 220 is placed on a bottom surface of the box 210. An opening at the top of the box 210 has a support on which the cell tray 100 is placeable. At this time, the non-woven fabric sheet 120 faces the inside of the box 210.

Then, the closed-end cylindrical recess 112 with its bottom surface being the non-woven fabric sheet 120 is formed at the top of the box 210. When the light emitting unit 220 emits illumination light, part of the illumination light passes through the non-woven fabric sheet 120 and the opening 111. Since the inside of the box 210 is matte black, the illumination light is not diffusely reflected in the box 210. As described above, the non-woven fabric sheet 120 has light transmittance when water is absorbed in it, and thus easily transmits the illumination light and absorbs little illumination light. When the cell aggregate is placed in the recess 112, part of the illumination light having passed through the non-woven fabric sheet 120 is blocked or attenuated by the cell aggregate. Specifically, when a camera is used to take an image of the cell aggregate and the recess 112, the illumination light blocked or attenuated by the cell aggregate is darker than the illumination light passing through only the non-woven fabric sheet 120, thereby providing high contrast between the cell aggregate and therearound. Thus, the cell aggregate can be easily recognized in the taken image. Specifically, the light emitted from below the non-woven fabric sheet 120 and having passed through the non-woven fabric sheet 120 should be recognized as a white circle, which is the shape of the opening 111, on an upper side of the non-woven fabric sheet 120, but the cell aggregate is in shadow and recognized as a nearly black circle. The black circular outline can be determined as an outline of the cell aggregate.

Next, with reference to Fig. 2, the cell structure producing apparatus 300 will be described. In Figs. 2 to 5, a direction from left to right is an X-axis appropriate direction, a direction from down to up is a Z-axis appropriate direction, and a direction from front to back is a Y-axis appropriate direction.

The cell structure producing apparatus 300 mainly includes a triaxial actuator 310, a camera (electronic camera) 320 including an imaging device as a light receiving unit, a piercing portion 330, and a control unit 340. The triaxial actuator 310 mainly includes an X-axis actuator 311, a Y-axis actuator 312, a Z-axis actuator 313, a securing portion 314, and a base 315. The light receiving unit is herein the electronic camera 320, but an optical sensor may be chosen as the light receiving unit. The piercing portion 330 mainly includes a chuck 331 and a needle 332 as a needle-shaped body extending long and thin. The base 315 is a seat arranged so that a surface of the base 315 is horizontal. Two axes crossing in the surface of the base 315 are defined as an X axis and a Y axis. The Y-axis actuator 312 is secured to the base 315, and supports the X-axis actuator 311 movably in the Y-axis direction relative to the base 315. A vertical direction perpendicular to the X axis and the Y axis is defined as a Z axis. In the piercing portion 330, the needle 332 is mounted to extend in the Z-axis direction. The X-axis actuator 311 supports the Z-axis actuator 313 movably in the X-axis direction relative to the base 315. The Z-axis actuator 313 supports the securing portion 314 movably in the Z-axis direction relative to the base 315. Thus, the triaxial actuator 310 and the needle 332 of the piercing portion 330 can be moved in directions of three axes: two horizontal axes and one vertical axis. The securing portion 314 holds the electronic camera 320 and the piercing portion 330. From the above configuration, the electronic camera 320 and the piercing portion 330 are movable in the X-axis, Y-axis, and Z-axis directions. The electronic camera 320 mainly includes an electronic imaging device and an imaging lens, and takes an image and transmits the image to the control unit 340. The chuck 331 receives and holds the needle 332 from a needle feeder (not shown). The needle feeder automatically feeds the needle 332 to the chuck 331. Thus, the needle 332 is automatically mounted to the chuck 331. The needle 332 is a long and thin needle-shaped body made of a material with cell nonadhesiveness, rust resistance, and a low elution property, for example, stainless or tungsten and having, for example, a conical tip. The needle 332 has rigidity enough to pierce the cell aggregate. A section of the needle 332 has any diameter such that the needle 332 does not break the cell aggregate when piercing the cell aggregate and does not prevent fusion of the cell aggregates, for example, 50 to 300 micrometers. The cell nonadhesiveness refers to a property of preventing adhesion of cells via an extracellular adhesion factor. A material with a low elution property has low cytotoxicity. The control unit 340 is electrically connected to the triaxial actuator 310, the electronic camera 320, and the piercing portion 330 and controls operations thereof. Generally stated, the control unit 340 drives the triaxial actuator 310 to move the electronic camera 320 onto the cell tray 100 and cause the electronic camera 320 to take an image of the cell aggregate placed in the recess 112. Then, the control unit 340 uses the image received from the electronic camera to calculate a position of the cell aggregate. The control unit 340 drives the needle 332 according to the calculated position and causes the needle 332 to pierce the cell aggregate. Specifically, the control unit 340 can calculate a center position of the cell aggregate from a black outline of the cell aggregate imaged on the upper side of the non-woven fabric sheet 120, and determine the position as a position to be stuck.

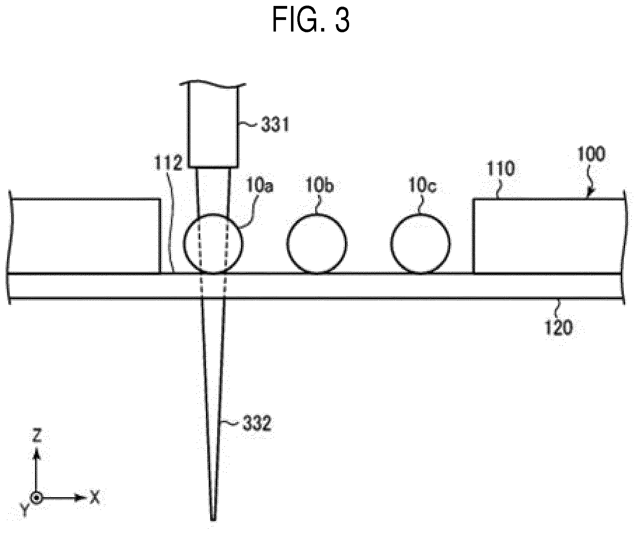

Next, with reference to Figs. 3 to 5, a process of the needle 332 piercing the plurality of cell aggregates will be described in detail.

First, a pipettor (not shown) is used to arrange the cell aggregates in the plurality of recesses 112 together with a buffer solution or a culture solution. At this time, the plurality of cell aggregates are placed in one recess 112. As described above, the non-woven fabric sheet 120 is made of a material that can hold the buffer solution or the culture solution, and the buffer solution or the culture solution has surface tension. Thus, the cell aggregates are covered by the buffer solution or the culture solution in the recess 112 due to the surface tension of the buffer solution or the culture solution held by the non-woven fabric sheet 120. Since the buffer solution or the culture solution contains nutrients or oxygen, the cell aggregates in the recess 112 are unlikely to be killed.

Next, the light emitting unit 220 emits illumination light to illuminate the non-woven fabric sheet 120. Specifically, the light emitting unit 220 emits light toward the cell aggregates. Then, the control unit 340 drives the triaxial actuator 310 to move the electronic camera 320 onto the cell tray 100. At this time, the non-woven fabric sheet 120 holds the buffer solution or the culture solution and thus has light transmittance. Thus, the illumination light easily passes through the non-woven fabric sheet 120. Then, part of the illumination light having passed through the non-woven fabric sheet 120 is blocked or attenuated by the cell aggregates. Thus, when the electronic camera 320 takes an image of the plurality of cell aggregates placed in the recess 112, the taken image has high contrast between the cell aggregates and therearound. The electronic camera 320 transmits the taken image to the control unit 340. The control unit 340 uses the taken image to detect each of the plurality of cell aggregates placed in the recess 112 and detect a position of each of the plurality of cell aggregates, particularly, a position in each of the cell aggregates to be stuck by the needle 332. The control unit 340 calculates a positional relationship between one particular cell aggregate 10a and the needle 332 based on the detected position, and obtains a drive amount of the needle 332 based on the calculated positional relationship. The triaxial actuator 310 drives the needle 332 based on the drive amount obtained by the control unit 340, and moves the needle 332 directly above the cell aggregate 10a in the recess 112. Then, the Z-axis actuator 313 moves the needle 332 down to the cell aggregate 10a along the Z axis.

When the tip of the needle 332 comes into contact with the cell aggregate 10a, the cell aggregate 10a is about to be deformed to be crushed, rotated, and/or moved by pressure of the tip. At this time, the non-woven fabric sheet 120 slightly bends and deforms so as to support a bottom surface of the cell aggregate 10a against the pressure of the tip and wrap around the cell aggregate 10a. Thus, the tip of the needle 332 reliably catches the cell aggregate 10a, and the needle 332 reliably sticks and penetrates the cell aggregate 10a at a position to be stuck. If the needle 332 is further moved down by a predetermined length, the tip of the needle 332 enters the non-woven fabric sheet 120. As described above, the non-woven fabric sheet 120 is made of a material that the needle 332 can easily penetrate without being damaged, in other words, the material and the structure of the non-woven fabric sheet 120 have low mechanical stress on the needle 332. Thus, the needle 332 can pierce the cell aggregate 10a over a desired length without the tip thereof being folded or bent when entering the non-woven fabric sheet 120.

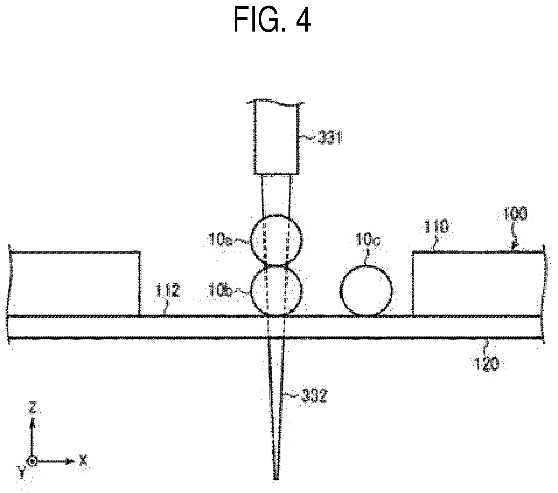

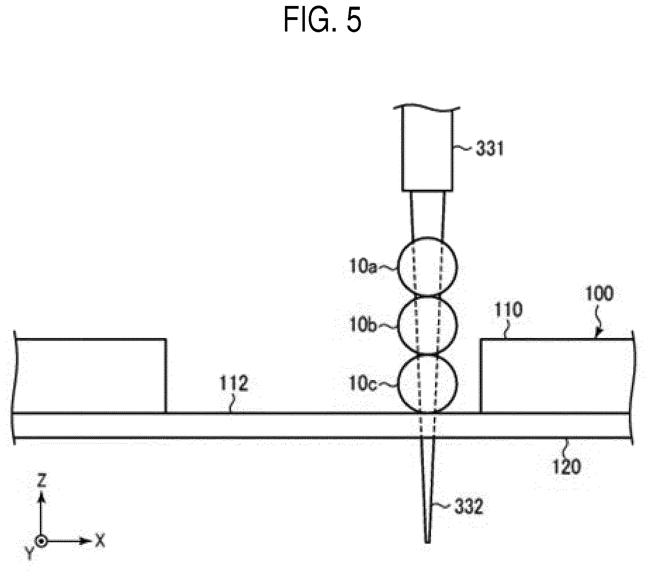

After the needle 332 is moved down by the predetermined length, the Z-axis actuator 313 moves the needle 332 up along the Z axis. At this time, the needle 332 is piercing through the cell aggregate 10a. Then, the control unit 340 and the triaxial actuator 310 again perform the same processes as described above. Thus, the needle 332 is moved directly above a next cell aggregate 10b and sticks and penetrates the next cell aggregate 10b (see Fig. 4). These processes are repeated for the number of the cell aggregates in the recess 112 to cause the needle 332 to penetrate all the cell aggregates in the recess 112 (see Fig. 5). An amount of downward movement of the needle 332 to the cell aggregate is determined according to a size of the cell aggregate and the number of the cell aggregates to be stuck, in other words, a position of the cell aggregate on the needle 332. Specifically, when the needle 332 sticks and penetrates the first cell aggregate, the amount of downward movement is the largest, and for the next cell aggregate, the amount of downward movement is slightly smaller than a diameter of the cell aggregate. Slightly reducing the amount of downward movement causes a close contact between the cell aggregates and facilitates fusion. These processes are repeated for the plurality of recesses 112 and the plurality of cell aggregates to obtain a plurality of needles 332 piercing through the plurality of cell aggregates. The amount of downward movement may be determined so that the amount of downward movement for the first cell aggregate is smaller than that in Fig. 3, that is, the first cell aggregate is stuck shallowly, and a second cell aggregate pierced thereafter further moves the first cell aggregate. After the needle 332 penetrates a desired number of cell aggregates, the plurality of needles 332 piercing through the cell aggregates are arranged so that the stuck cell aggregates form a desired three-dimensional shape and moved to an aftertreatment module (not shown). The aftertreatment module is a so-called perfusion culture container that holds the plurality of needles piercing through the cell aggregates, and perfuses the buffer solution or the culture solution to the cell aggregates. Since the buffer solution or the culture solution contains nutrients or oxygen, the cell aggregates can fuse together without being killed. If the needles 332 are drawn from the cell aggregates after a lapse of a predetermined time, a three-dimensional structure of cells can be obtained.

According the present invention, when the needle-shaped body sticks and penetrates the cell aggregates, the needle-shaped body can pierce the cell aggregates without being deformed or broken, thereby obtaining a three-dimensional structure of cells of any shape.

In the above described embodiment, the triaxial actuator 310 moves the electronic camera 320 and the piercing portion 330 relative to the cell tray 100. However, the cell tray 100 may be moved relative to the electronic camera 320 and the piercing portion 330. The electronic camera 320 and the piercing portion 330 as well as the cell tray 100 may be moved relative to each other.

The color of the non-woven fabric sheet 120 is not limited to the above described color, but the non-woven fabric sheet 120 may have light transmittance when liquid is absorbed in it such as the buffer solution or the culture solution. The non-woven fabric sheet 120 may have light transmittance both when water is absorbed or not absorbed in it. The fibrous material for the non-woven fabric sheet 120 may be made of, for example, engineering plastic such as polypropylene, nylon, polyester, polyethylene (PE), polyacetal (POM), polycarbonate (PC), polyacrylonitrile (PAN), polyetheretherketone(PEEK), monomer casting nylon (MCN), 6 nylon (6N), or 66 nylon (66N).

The matrix of the openings 111 is not limited to the 8-by-12 matrix, but may be a matrix of other numbers.

The box 210 may be made of black resin, and the inside of the box 210 may be blasted to be matte black.

The light emitted from the light emitting unit 220 and the LED 222 is not limited to the white light, but the emitted light may have a wavelength suitable for detecting the position of the cell aggregate by image analysis. The color of the light transmitting plate 221 is not limited to white, but the light transmitting plate 221 may transmit light having a wavelength suitable for detecting the position of the cell aggregate by image analysis.

An inner surface of the frame 110 when mounted to the cell tray rack 200 may be painted matte black, or the frame 110 made of a black material may be blasted to be matte black. The materials for the frame 110 and the box 210 are not limited to those described above, but may include, not limited to, resin such as polypropylene, nylon, materials coated with fluorine, Teflon(R), poly-HEMA, acrylic plate, vinyl chloride plate, polyester resin plate, or polycarbonate plate, or engineering plastic such as polypropylene(PP), acrylonitrile butadiene styrene (ABS), polyethylene(PE), polyacetal (POM), polycarbonate (PC), polyetheretherketone (PEEK), monomer casting nylon (MCN), 6 nylon (6N), or 66 nylon (66N) as long as the material is surface-treated to be matte black or the black material is blasted to be matte black to prevent the illumination light from being diffusely reflected. The method for forming the frame 110 and the box 210 is not limited to the above described method, but the frame 110 and the box 210 may be formed by other methods.

The material for the needle 332 is not limited to the above described material, but may include, not limited to, other materials with cell nonadhesiveness, rust resistance, and a low elution property. Besides, materials with low cell adhesiveness may be used. The material for the needle 332 may be a material without cell nonadhesiveness, rust resistance, and a low elution property, and may be a material having rigidity enough to pierce the cell aggregate and/or a material machined to have an extremely small diameter of, for example, about 170 micrometers.

The needle 332 needs not penetrate over the entire length of the opening 111. Specifically, the tip of the needle 332 may enter the opening 111 up to half the entire length.

A plurality of needles 332 may be simultaneously used. Specifically, the plurality of needles 332 simultaneously pierce the cell aggregates. This can reduce time required for piercing all the cell aggregates. At this time, a center-to-center distance between the adjacent recesses 112 may be equal to a center-to-center distance between adjacent needle-shaped bodies.

The shape of the opening 111 is not limited to the cylindrical shape, but may be a rectangular shape, an elliptical shape, or other shapes. Two ends of the opening 111 need not have the same shape, but the opening 111 may extend through the frame 110.

In the above description, the cell structure producing apparatus 300 includes the triaxial actuator 310. However, the cell structure producing apparatus 300 may include an actuator or a robot that can be driven at least in directions of three axes, for example, a robot having three or more axes such as a SCARA robot or a vertical articulated robot rather than the triaxial actuator 310.

The chuck 331 needs not receive the needle 332 from the needle feeder, but may receive the needle 332 from a holder that holds the plurality of needles 332 at regular intervals or other members. The tip of the chuck 331 may be automatically opened/closed, the needle feeder may feed needles one by one to the chuck 331 at a fixed position, and the chuck 331 may automatically open the tip to receive the needle at the fixed position and then close the tip.

The sizes of the members in the specification and the drawings are exemplary and not limited to them. The materials for the members are exemplary and not limited to them.

The plurality of embodiments of the present invention have been described with reference to the accompanying drawings. However, it is apparent to those skilled in the art that modifications may be made in the structures and relationships of the components without departing from the scope and spirit of the described invention.

This application claims priority to Japanese Patent Application No. 2016-241454 filed on December 13, 2016, which is incorporated by reference.

### Reference Signs List

- 100: cell tray
- 110: frame
- 111: opening
- 112: recess
- 120: non-woven fabric sheet
- 200: cell tray rack
- 210: box
- 220: light emitting unit
- 300: cell structure producing apparatus
- 310: triaxial actuator
- 311: X-axis actuator
- 312: Y-axis actuator
- 313: Z-axis actuator
- 314: securing portion
- 315: base
- 320: electronic camera
- 330: piercing portion
- 340: control unit

## Claims

1. A cell structure producing apparatus for sticking and penetrating a cell aggregate held on a cell tray, comprising a movable needle-shaped body,
wherein the cell tray includes a frame having an opening, and a porous member supported in the opening by the frame, the cell aggregate being placeable on the porous member, and
wherein a tip of the needle-shaped body is capable of penetrating through the porous member when the needle-shaped body sticks and penetrates the cell aggregate.

2. A cell structure producing apparatus according to claim 1, comprising a light emitting unit configured to emit light from one side of the porous member, and a light receiving unit configured to receive the light on another side of the porous member,
wherein the porous member transmits the light emitted from the one side when liquid is absorbed in the porous member.

3. A cell structure producing apparatus according to claim 2,
wherein the light receiving unit is a camera configured to take an image of the opening.

4. A cell structure producing apparatus according to any one of claims 1 to 3, wherein the porous member is a fiber sheet.

5. A cell structure producing apparatus according to claim 4,
wherein the fiber sheet is a non-woven fabric.

6. A cell structure producing apparatus according to claim 5,
wherein the frame is in a form of a plate, and the opening is a through hole extending through the plate in a thickness direction of the plate.

7. A cell structure producing apparatus according to claim 6,
wherein the non-woven fabric is supported by the frame so as to close the through hole.

8. A cell tray for holding a cell aggregate in a cell structure producing apparatus including a movable needle-shaped body, for sticking and penetrating the cell aggregate by the movable needle-shaped body, the cell tray comprising:
a frame having an opening; and
a porous member supported in the opening by the frame, the cell aggregate being placeable on the porous member,
wherein a tip of the needle-shaped body is capable of penetrating through the porous member when the needle-shaped body sticks and penetrates the cell aggregate.

9. A cell tray according to claim 8,
wherein the cell structure producing apparatus includes a light emitting unit configured to emit light from one side of the porous member, and a light receiving unit configured to receive the light on another side of the porous member, and
wherein the porous member transmits the light emitted from the one side when liquid is absorbed in the porous member.

10. A cell tray according to claim 9, wherein the light receiving unit is a camera configured to take an image of the opening.

11. A cell tray according to any one of claims 8 to 10,
wherein the porous member is a fiber sheet.

12. A cell tray according to claim 11, wherein the fiber sheet is a non-woven fabric.

13. A cell tray according to claim 12, wherein the frame is in a form of a plate, and the opening is a through hole extending through the plate in a thickness direction of the plate.

14. A cell tray according to claim 13, wherein the non-woven fabric is supported by the frame so as to close the through hole.
